# EUROPÄISCHE PATENTSCHRIFT

(11) **EP 1 277 492 B1**
(45) Veröffentlichungstag und Bekanntmachung des Hinweises auf die Patenterteilung: **18.10.2006**
(21) Anmeldenummer: 02090260.7
(22) Anmeldetag: 16.07.2002
(51) Int. Cl.: A61N 1/368

(54) **Zweikammer-Herzschrittmacher**
Dual chamber pacemaker
Stimulateur cardiaque double chambre

(30) Priorität: 20.07.2001 DE 10136641
(43) Veröffentlichungstag der Anmeldung: 22.01.2003
(73) Patentinhaber: BIOTRONIK GmbH & Co. KG, 12359 Berlin (DE)
(72) Erfinder: Busch, Ulrich, 10318 Berlin (DE); Schaldach, Max, verstorben (DE)
(74) Vertreter: Lindner-Vogt, Karin L.

(56) Entgegenhaltungen:
- US-A- 4 788 980

## Beschreibung

Die Erfindung betrifft einen Zweikammer-Herzschrittmacher mit einer atrialen Detektionseinheit, die zum Erfassen elektrischer Signale im Atrium eines Herzens als atriales Sense-Ereignis ausgebildet ist, einer ventrikulären Detektionseinheit, die zum Erfassen elektrischer Signale eines Ventrikels des Herzens als ventrikuläres Sense-Ereignis ausgebildet ist, einer Stimulationseinheit, die zumindest zum Auslösen der Abgabe elektrischer Impulse an den Ventrikel als ventrikuläre Stimulationsereignisse ausgebildet ist, sowie einer Steuereinheit, die mit der atrialen und der ventrikulären Detektionseinheit sowie der Stimulationseinheit verbunden und ausgebildet ist, das Auslösen der Impulsabgabe in Abhängigkeit von der atrialen Detektionseinheit und der ventrikulären Detektionseinheit so zu steuern, dass eine Impulsabgabe an den Ventrikel nach Ablauf einer AV-Zeit nach Erfassen eines atrialen Senseereignisses ausgelöst wird, falls vor Ablauf der AV-Zeit kein ventrikuläres Sense-Ereignis durch die ventrikuläre Detektionseinheit erfasst wird, wobei die Steuereinheit weiterhin ausgebildet ist, ventrikuläre Sense-Ereignisse in mindestens zwei Kategorien einzuteilen, von denen eine erste Kategorie einer natürlichen Reizleitung vom Atrium zum Ventrikel zuzuordnende ventrikuläre Sense-Ereignisse betrifft, während eine zweite Kategorie ventrikulären Extrasystolen oder vorzeitigen ventrikulären Kontraktionen (PVC) zuzuordnende ventrikuläre Sense-Ereignisse betrifft und die Steuerung des Herzschrittmachers danach differenziert wird, ob ein ventrikuläres Sense-Ereignis der ersten Kategorie zugeordnet wird, oder nicht.

Herzschrittmacher werden üblicherweise mit drei Buchstaben bezeichnet, von denen der erste Buchstabe die stimulierte Kammer, der zweite Buchstabe die wahrnehmende Kammer (Sensing) und der dritte Buchstabe einen Schrittmacherbetriebsmodus bezeichnet. Die vorliegende Erfindung betrifft in erster Linie VDD- oder DDD-Schrittmacher. VDD-Schrittmacher sind geeignet, den Ventrikel eines Herzens zu stimulieren, elektrische Signale sowohl im Ventrikel als auch im Atrium aufzunehmen und sowohl in einem inhibierten als auch in einem getriggerten Modus zu arbeiten. DDD-Schrittmacher sind zusätzlich in der Lage, auch das Atrium eines Herzens zu stimulieren.

Bei solchen Schrittmachern ist üblicherweise eine atriale Refraktärzeit vorgesehen, in der elektrische Signale im Atrium eines Herzens entweder gar nicht detektiert werden, oder detektierte Signale nicht weiter verarbeitet werden. Typische, zu detektierende Signale sowohl im Atrium als auch im Ventrikel eines Herzens sind atriale bzw. ventrikuläre Kontraktionen des Herzens, die mit einer Depolarisation des Herzgewebes einhergehen, welche elektrisch detektierbar ist.

Auf ein detektiertes und verarbeitetes elektrisches Signal im Atrium eines Herzens wird üblicherweise eine AV-Zeit ausgelöst, zu deren Ende ein elektrischer Stimulationsimpuls an den Ventrikel des Herzens abgegeben wird, falls nicht während der AV-Zeit eine natürliche Kontraktion des Ventrikels detektiert wird.

Die zeitliche Abstimmung einer atrialen Kontraktion, auf die eine ventrikuläre Kontraktion folgt, ist von großer Bedeutung für die hämodynamische Leistung, also die Pumpleistung eines Herzens. Zunächst erfolgt eine Kontraktion des Atriums, auf die im bestimmten Abstand eine Kontraktion eines Ventrikels folgt. Nach einer weiteren Zeit, der VA-Zeit ist wieder eine Kontraktion des Atriums fällig, auf die nach einer weiteren AV-Zeit wieder eine Kontraktion des Ventrikels folgt und so fort.

Bei der Stimulation insbesondere des Ventrikels ist es zu vermeiden, dass diese nicht allzu schnell auf eine vorangehende natürliche oder stimulierte Kontraktion des Ventrikels folgt, da es aufgrund der elektrischen Reizleitung im Herzgewebe (Myokard) ansonsten zu einem Kammerflimmern des Ventrikels kommen kann, welches einen Ausfall der Pumpleistung des Herzens zur Folge hat und tödlich enden kann, sofern es nicht rechtzeitig beendet wird.

Weiterhin ist es möglich, dass ventrikuläre Stimulationsereignisse oder auch natürliche Kontraktionen des Ventrikels über eine elektrische Fernfeldwirkung im Atrium als elektrische Signale wahrgenommen werden. Eine solche Fernfeldwahrnehmung kann ebenfalls zu einer Schrittmacher-vermittelten Tachykardie führen.

Schließlich kann es auch zu einer retrograden (rückwärts gerichteten) Reizleitung vom Ventrikel zum Atrium kommen, die eine vorzeitige Kontraktion des Atriums zur Folge hat (PAC: premature atrial contraction). Wenn ein Herzschrittmacher auf Grund einer solchen vorzeitigen atrialen Kontraktion das AV-Intervall startet und zu dessen Ende eine ventrikuläre Stimulation auslöst, kann diese wiederum zu einer weiteren PAC führen, so dass es zu einer Schrittmacher-verursachten Tachykardie, einer überhöhten Herzrate kommt.

Um dies zu verhindern, ist bei Schrittmachern üblicherweise eine postventrikuläre atriale Refraktärzeit (PVARP=Postventricular Atrial Refractary Period) vorgesehen. Dieses Intervall wird nach einem ventrikulären Stimulationsereignis ausgelöst und hat die Wirkung, dass während des PVARP-Intervalls wahrgenommene atriale Sense-Ereignisse nicht zum Auslösen eines AV-Intervalls weiterverarbeitet werden. Es ist bekannt, das PVARP-Intervall nach vorzeitigen ventrikulären Kontraktionen (PVC) oder ventrikulären Extrasystolen (VES) zu verlängern, um eine schrittmacherverursachte Tachykardie (PMT) zu vermeiden.

Es ist möglich, dass natürliche atriale Ereignisse insbesondere während einer verlängerten PVARP nicht wahrgenommen werden. Die Folge ist, dass eine natürliche ventrikuläre Kontraktion, die auf eine nicht wahrgenommene atriale Kontraktion erfolgt, nicht als natürliche ventrikuläre Kontraktion, sondern als ventrikuläre Extrasystole wahrgenommen wird, und so fort. Dieses Phänomen ist als VES-Lockin bekannt. VES-Lockin kann sowohl im VDDoder auch im DDD-Betrieb bei Schrittmachern mit herkömmlichem atrialen Refraktärzeitkonzept (relativ lange PVARP) vornehmlich bei höheren Stimulationsraten auftreten, falls ein Patient zumindest temporär eine relativ langsame Überleitung atrialer Reize zum Ventrikel (AV-Überleitung) hat.

Ein VES-Lockin entsteht, wenn ein vorzeitiges atriales Sense-Ereignis in eine atriale Refraktärzeit nach einem ventrikulären Ereignis fällt zum Beispiel im Falle von
- vorzeitigen atrialen Kontraktionen mit Sinusknotenreset während PVARP nach einem ventrikulären Stimulationsereignis
- vorzeitige atriale Kontraktion mit Sinusknotenreset während der PVARP nach einem ventrikulären Sense-Ereignis
- vorzeitige oder reguläre atriale Kontraktion während einer verlängerten PVARP nach einer vorzeitigen ventrikulären Kontraktion (der oben geschilderte Fall)
- wenn sich ein Schrittmacher im Wenckebach-Zustand befindet, bei dem die ventrikuläre Stimulation nicht am Ende des AV-Intervalls erfolgt, wenn zu diesem Zeitpunkt das mit allen ventrikulären Sense- und Stimulationsereignissen gestartete "upper tracking intervall" noch nicht beendet ist, sondern die ventrikuläre Stimulation an das Ende des "upper tracking intervalls" verschoben wird. Hier ist es aufgrund der häufigen Asynchronizität von atrialen und ventrikulären Ereignissen möglich, daß ein reguläres atriales Sense-Ereignis in die PVARP (postventrikuläre atriale Refraktärzeit) nach ventrikulärer Stimulation fällt und (langsam) natürlich übergeleitet wird. Eine solche auf natürlicher Überleitung beruhende ventrikuläre Kontraktion wird dann als ventrikuläre Extrasystole VES klassifiziert.

Neben den genannten Ursachen für ein VES-Lockin gibt es Weitere.

Im Zusammenhang mit dem Verhindern von schrittmacherverursachten Tachykardien (PMT) und der dazu dienenden Verlängerung der PVARP nach einer ventrikulären Extrasystole passiert es, dass eine vorzeitige atriale Kontraktion oder ein atriales Sense-Ereignis im Herzen langsam vom Atrium zum Ventrikel übergeleitet wird und im Ventrikel nach Ablauf der im Schrittmacher vorgesehenen Überleitungszeit eine ventrikuläre Depolarisation und Kontraktion des Ventrikels verursacht, die dann vom Schrittmacher als ventrikuläre Extrasystole klassifiziert wird. Dies wiederum hat eine Verlängerung der PVARP zur Folge.

Die nächste antegrade Erregung des Atriums fällt in die verlängerte PVARP und löst durch natürliche Reizleitung vom Atrium zum Ventrikel die nächste Kontraktion des Ventrikels aus, die wiederum als ventrikuläre Extrasystole klassifiziert wird. Dieses Verhalten des Herzschrittmachers stellt sich nach außen wie ein temporärer Verlust der Detektion atrialer Ereignisse, des atrialen Sensings, dar.

Für den Patienten bedeuten längere AV-Zeiten eine Verschlechterung der Ventrikelfüllung, da sich die Klappen zwischen Atrium und Ventrikel frühzeitig verschließen. Dies ist bei einigen Patienten problematisch. Außerdem kann die Depolarisierung des Atriums (P-Welle) bei höheren Frequenzen in eine ventrikuläre Systole fallen, mit der Folge, dass Patienten unter Vorhofpfropungen, die eine potentielle Flimmergefahr birgen, wie bei einem Schrittmachersyndrom leiden.

Ein solches VES-Lockin wird erst dann beendet, wenn das spontane (natürliche) atriale Intervall länger als das von einer atrialen Depolarisation (P-Welle) zu einer ventrikulären Depolarisation (Q-Zacke) reichende PQ-Intervall plus dem PVARP ist oder wenn ein mit einer ventrikulären Extrasystole gestarteter Maintimer eines Herzschrittmachers vor dem nächsten ventrikulären Sense-Ereignis abläuft.

Der Erfindung liegt die Aufgabe zu Grunde, eine möglichst weitgehende Abhilfe bezüglich der zuvor geschilderten Probleme zu schaffen.

Erfindungsgemäß wird diese Aufgabe durch einen Zweikammer-Herzschrittmacher der eingangs genannten Art gelöst, bei welchem für die Steuerung des Auslösens der Impulsabgabe ein AV-Überwachungsintervall derart vorgesehen ist, dass das AV-Überwachungsintervall von jedem atrialen Sense-Ereignis ausgelöst wird, welches außerhalb einer atrialen Refraktärzeit liegt, wobei der Herzschrittmacher ausgebildet ist, innerhalb des AV-Überwachungsintervalls auftretende ventrikuläre Ereignisse der ersten Kategorie (auf natürlicher Reizleitung beruhende ventrikuläre Kontraktion) zuzuordnen. Ein hiervon unabhängiger, aber grundsätzlich synergetisch wirkender Lösungsaspekt besteht darin, dass für die Steuerung des Auslösens der Impulsabgabe ein Fernfeldschutzintervall sowie ein PMT-Schutzintervall vorgesehen sind,
- derart, dass das Fernfeldschutzintervall von jedem ventrikulären Stimulationserereignis und von jedem von der ventrikulären Detektionseinheit erfassten ventrikulären Sense-Ereignis ausgelöst wird, wobei das Fernfeldschutzintervall hinsichtlich seiner Zeitdauer so bemessen ist, dass ein atriales Sense-Ereignis infolge einer Fernfeldwirkung eines ventrikulären Ereignisses in das Fernfeldschutzintervall fällt, und wobei das Fernfeldschutzintervall die Wirkung hat, dass während des Fernfeldschutzintervalls von der atrialen Detektionseinheit erfasste atriale Ereignisse für das Auslösen der Impulsabgabe durch die Stimulationseinheit unberücksichtigt bleiben, so dass ein Auslösen der Impulsabgabe nach einem atrialen Sense-Ereignis infolge von Cross-Sensing oder Fernfelderfassung ventrikulärer Ereignisse unterbleibt sowie
- derart, dass das PMT-Schutzintervall von jedem ventrikulären Stimulationsereignis und von jedem, von der ventrikulären Detektionseinheit erfassten ventrikulären Sense-Ereignis ausgelöst wird, wenn dieses ventrikuläre Ereignis außerhalb einer vorgebenen AV-Zeit oder außerhalb eines AV-Überwachungsintervalls erfasst wurde, wobei sich das PMT-Schutzintervall, falls es ausgelöst wurde, an das jeweilige Fernfeldschutzintervall anschließt und dieses verlängert und wobei das PMT-Schutzintervall die Wirkung hat, dass während des PMT-Schutzintervalls von der atrialen Detektionseinheit erfasste atriale Ereignisse für das Auslösen der Impulsabgabe durch die Stimulationseinheit unberücksichtigt bleiben, so dass ein Auslösen der Impulsabgabe infolge eines atrialen Ereignisses, welches auf eine retrograde Reizleitung vom Ventrikel zum Atrium zurückgeht, unterbleibt.

Das Konzept der konventionellen atrialen Refraktärzeit, die gegen auf Fernfeldwirkung ventrikulärer Ereignisse beruhende atriale Sense-Ereignisse und retrograde Reizleitung vom Ventrikel zum Atrium gleichermaßen schützen soll, ist somit bei dem erfindungsgemäßen Herzschrittmacher einerseits durch eine Differenzierung der auf ein ventrikuläres Ereignis folgenden atrialen Refraktärzeit in ein Fernfeldschutzintervall und ein daran anschließendes PMT-Schutzintervall realisiert. Außerdem wird das AV-Überwachungsintervall neu eingeführt, das eine verbesserte Klassifizierung von auf natürlicher Reizleitung beruhenden ventrikulären Ereignissen einerseits und PVC's bzw. VES' andererseits ermöglicht. Das Fernfeldschutzintervall startet mit jedem nicht refraktären ventrikulären Ereignisses. Es ist gerade so lang bemessen, dass das Atrium vor einer Fernfeldwirkung eines ventrikulierenden Ereignisses ausreichend geschützt ist. Atriale Ereignisse innerhalb des Fernfeldschutzintervalls bleiben für die weitere Schrittmachersteuerung unberücksichtigt.

Das PMT-Schutzintervall startet dagegen ausschließlich nach ventrikulären Stimulations-Ereignissen oder solchen Sense-Ereignissen, die außerhalb der AV-Zeit oder außerhalb des AV-Überwachungsintervalls liegen, d. h. nur nach ventrikulären Stimulationsereignissen und solchen ventrikulären Sense-Ereignissen, die der zweiten Kategorie zugeordnet werden und entsprechend als VES bzw. PVC kategorisiert werden. Während des PMT-Schutzintervalls wird verhindert, dass retrograde Reizleitungen vom Ventrikel zum Atrium eine AV-Überleitung der Reize vom Atrium zum Ventrikel starten oder einen Maintimer des Herzschrittmachers zurücksetzen können. Atriale Sense-Ereignisse innerhalb des PMT-Schutzintervalls und außerhalb der atrialen Refraktärzeit werden jedoch als Sense-Ereignisse wahrgenommen und für die weitere Herzschrittmachersteuerung weiterverarbeitet.

Das bereits angesprochene und auch unabhängig von dem Fernfeldschutzintervall und dem PMT-Schutzintervall vorzusehende AV-Überwachungsintervall wird mit allen atrialen Sense-Ereignissen außerhalb einer atrialen Refraktärzeit und - sofern vorhanden - des Fernfeldschutzintervalls gestartet. Sämtliche, sowohl auf antegrader Reizleitung als auch auf retrograder Reizleitung beruhenden Depolarisationen, auch solche in einem PMT-Schutzintervall, starten das AV-Überwachungsintervall. Das AV-Überwachungsintervall wird von ventrikulären Stimulationsereignissen und ventrikulären Sense-Ereignissen der ersten Kategorie (auf natürlicher Reizüberleitung beruhend) wieder gelöscht.

Bei einem bevorzugten Herzschrittmacher sind sowohl ein atriales Überwachungsintervall als auch ein Fernfeldschutzintervall und ein PMT-Schutzintervall vorgesehen. Fällt bei einem solchen Herzschrittmacher eine atriale Depolarisation, also ein atriales Sense-Ereignis, in das PMT-Schutzintervall, so wird das nächste ventrikuläre Ereignis innerhalb des AV-Überwachungsintervalls als natürliches ventrikuläres Sense-Ereignis der ersten Kategorie und nicht wie herkömmlich, als PVC oder VES klassifiziert. Ein solches ventrikuläres Sense-Ereignis der ersten Kategorie startet im Gegensatz zu einer PVC oder einer VES kein PMT-Schutzintervall und das nächste atriale Sense-Ereignis wird als für die Steuerung weiter zu verarbeitendes atriales Sense-Ereignis erfasst und kann eine AV-Überleitung starten. Auf diese Weise wird das zuvor beschriebene VES-Lockin verhindert.

Da das AV-Überwachungsinterval mit allen atrialen Sense-Ereignissen außerhalb der atrialen Refraktärzeit gestartet wird, wird im Wenckebach-Betrieb eine ventrikuläre Wahrnehmung, die innerhalb des upper tracking intervalls und außerhalb des AV-Intervalls liegt, als reguläres ventrikuläres Sense-Ereignis und nicht als VES interpretiert, solange das AV-Überwachungsinterval noch andauert. Damit wird ein VES-Lockin hier ebenfalls verhindert.

Vorteilhafterweise weist ein Herzschrittmacher, bei dem ein AV-Überwachungsintervall vorgesehen ist, eines oder mehrere der folgenden Merkmale auf, unabhängig davon, ob bei dem Herzschrittmacher auch ein Fernfeldschutzintervall oder ein PMT-Schutzintervall vorgesehen ist:
- Eine postventrikuläre atriale Refraktärzeit (PVARP) wird verlängert, falls ein ventrikuläres Sense-Ereignis der zweiten Kategorie zuzuordnen ist und wird unverlängert belassen, falls das ventrikuläre Sense-Ereignis der ersten Kategorie zuzuordnen ist.
- Der Herzschrittmacher, insbesondere dessen Steuereinheit, ist ausgebildet, im Falle innerhalb des AV-Überwachungsintervalls auftretender ventrikulärer Sense-Ereignisse ein VA-Intervall zu starten, welches vorzeitig beendet wird, falls vor dessen Ablauf ein atriales Sense-Ereignis auftritt. Dabei ist diese Herzschrittmacher und insbesondere dessen Steuereinheit vorzugsweise so ausgebildet, dass er mit einem atrialen Sense-Ereignis ein AA-Intervall startet, welches vorzeitig beendet wird, falls innerhalb des AV-Überwachungsintervalls ein ventrikuläres Sense-Ereignis auftritt und das VA-Intervall gestartet wird, oder falls vor Ablauf des AA-Intervalls ein atriales Sense-Ereignis auftritt. Im Zusammenhang mit diesen bevorzugten Ausführungsformen ist es besonders bevorzugt einen Herzschrittmacher mit einer atrialen Stimulationseinheit vorzusehen, die zum Auslösen der Abgabe elektrischer Impulse an das Atrium als atriale Stimulationsereignisse ausgebildet ist, wobei der Herzschrittmacher, insbesondere dessen Steuereinheit, ausgebildet ist, die Abgabe eines atrialen Stimulationsimpulses mit Ablauf des VA-Intervalls oder des AA-Intervalls auszulösen, falls das VA-Intervall oder das AA-Intervall nicht vorzeitig beendet werden.

Die Erfindung soll nun anhand eines Ausführungsbeispiels mit Hilfe den Figuren näher erläutert werden. Von den Figuren zeigt:
- Figur 1:: Ein schematisches Blockschaltbild der Steuerung eines Herzschrittmachers der erfindungsgemäßen Art.
- Figur 2:: Ein mit der Steuerung aus Figur 1 bewirktes Timing eines Herzschrittmachers.

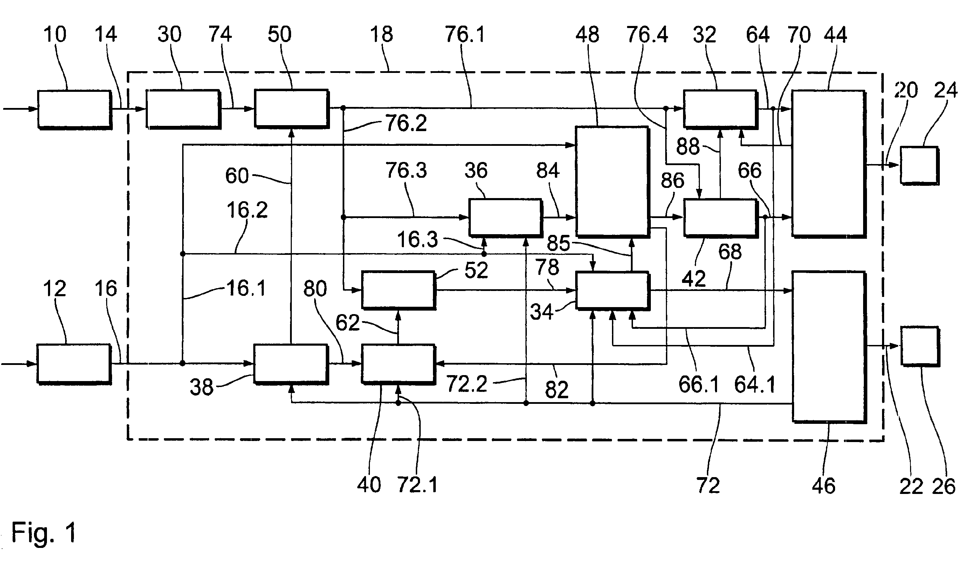

Die wesentlichen Bestandteile des in der Figur 1 abgebildeten Blockschaltbildes sind eine atriale Detektionseinheit 10 und eine ventrikuläre Detektionseinheit 12. Die Detektionseinheiten 10 und 12 sind beim Betrieb eines Herzschrittmachers mit Elektroden im Atrium bzw. im Ventrikel eines Herzens verbunden und ausgebildet, eine atriale bzw. eine ventrikuläre Depolarisation kennzeichnende elektrische Signale aufzunehmen und zu verarbeiten und entsprechende Steuersignale abzugeben, falls eine atriale oder eine ventrikuläre Depolarisation vorliegt. Diese Steuersignale werden über Signalleitungen 14 bzw. 16 an eine Steuereinheit 18 weitergegeben. Innerhalb dieser Steuereinheit 18 werden die Signale verarbeitet. Je nach Ablauf der Signalverarbeitung gibt die Steuereinheit 18 über Steuerleitungen 20 und 22 Steuersignale an eine atriale Stimulationseinheit 24 bzw. eine ventrikuläre Stimulationseinheit 26 ab. Die Stimulationseinheiten 24 und 26 sind mit im Atrium bzw. in Ventrikel des Herzens angeordneten Elektroden verbunden und geeignet ausgebildet, über diese Elektroden elektrische Impulse an das Myokard des Atriums bzw. des Ventrikels abzugeben und auf diese Weise eine Kontraktion des Atriums bzw. des Ventrikels zu stimulieren.

Im Folgenden soll nun die Steuereinheit 18 näher erläutert werden.

Allem voran sei bemerkt, dass eine Steuereinheit eines Herzschrittmachers über die Merkmale der Steuereinheit 18 hinaus weitere Merkmale aufweisen kann. Der Übersichtlichkeit halber sind bei der Steuereinheit 18 aus der Figur 1 all die Funktionseinheiten weggelassen, die beispielsweise der Überprüfung eines Stimulationserfolges, der sogenannten Capture-Control dienen, sowie Funktionseinheiten zur Erfassung statistischer oder medizinischer Taten des Patienten.

Wesentliche Einheiten der Steuereinheit 18 sind Timer, nämlich ein atrialer Refraktärzeittimer 30, ein AA-Timer (Maintimer) 32, ein AV-Timer 34, eine AV-Überwachungseinheit 36, eine Fernfeldschutzeinheit 38, eine PMT-Schutzeinheit 40 und ein VA-Timer 42. Neben diesen Timern sind eine atriale Stimulationssteuereinheit 44 und eine ventrikuläre Stimulationssteuereinheit 46 vorgesehen. Hinzu kommen eine V-Sense-Klassifikationseinheit 48 und ein Fernfeldschutzinhibitor 50 sowie ein PMT-Schutzinhibitor 52.

Allen Timern 30 bis 42 ist gemein, dass sie durch ein auslösendes Steuersignal ein jeweiliges Zeitintervall starten und je nach Art des Timers während dieses Zeitintervalls oder mit Ablauf des jeweiligen Zeitintervalls Signale ausgeben, bzw. die Weiterleitung von Signalen unterdrücken. Die Timer 32 - 42 können darüberhinaus über Reset-Signale zurückgesetzt werden.

Die beiden Inhibitoren 50 und 52 sind jeweils über eine Steuerleitung 60 bzw. 62 mit jeweils einen Timer, nämlich zum einen der Fernfeldschutzeinheit 38 und zum anderen der PMT-Schutzeinheit 40 verbunden. Die Inhibitoren 50 und 52 haben die Eigenschaft, die Weiterleitung von Signalen solange zu unterbrechen, solange über die Steuerleitungen 60 bzw. 62 ein entsprechendes Signal anliegt.

Die beiden Stimulationssteuereinheiten 44 und 46 sind so ausgebildet, dass sie zum einen auf entsprechende, an Steuerleitungen 64, 66 oder 68 anliegende Steuersignale hin über die Steuerleitungen 20 und 22 Steuerausgangssignale an die Stimulationseinheiten 24 bzw. 26 ausgeben und auf diese Weise eine Stimulation des Atriums bzw. des Ventrikels auslösen. Die Stimulationssteuereinheiten 44 und 46 umfassen weiterhin nicht näher dargestellte Capture-Control-Einheiten, die ausgebildet sind, ein Signal zu erzeugen, wenn die Stimulation des Atriums oder des Ventrikels über die Stimulationseinheit 24 bzw. 26 erfolgreich war. In diesem Fall geben die Stimulationssteuereinheiten 44 und 46 jeweils über Steuerrückleitungen 70 und 72 Signale aus.

Das Zusammenwirken der genannten Einheiten sowie die bisher nicht benannten Steuerleitungen sollen nun ausgehend vom Auftreten eines atrialen Sense-Ereignisses beschrieben werden.

Ein atriales Sense-Ereignis wird durch die atriale Detektionseinheit 10 erfasst, die daraufhin ein Steuersignal über die Steuerleitung 14 an den atrialen Refraktärzeittimer 30 abgibt. In diesem wird nun ein atriales Refraktärintervall gestartet und gleichzeitig ein Ausgangssignal über eine Steuerleitung 74 und durch die Fernfeldschutzeinheit 50 hindurch über Steuerleitungen 76.1, 76.2 und 76.3 an den AA-Timer 32, die AV-Überwachungseinheit 36 sowie durch den PMT-Schutzinhibitor 52 und über die Steuerleitung 78 an den AV-Intervalltimer 34 weitergeleitet.

Der AA-Timer, die AV-Überwachungseinheit 36 sowie der AV-Intervalltimer 34 werden auf diese Weise durch ein atriales Sense-Ereignis gestartet.

Solange das atriale Refraktärzeitintervall des atrialen Refraktärzeittimers 30 läuft, wird ein an der Steuerleitung 14 anliegendes, ein atriales Sense-Ereignis kenn- zeichnendes Signal nicht weitergeleitet. Ein die atriale Refraktärzeit auslösendes atriales Sense-Ereignis wird über die Steuerleitung 74 weitergeleitet, gelangt jedoch nur dann zu den Steuerleitungen 76.1, 76.2 und 76.3, falls an dem Fernfeldschutzinhibitor 50 kein entsprechendes Steuersignal über die Steuerleitung 60 anliegt. In gleicher Weise wird ein, ein atriales Sense-Ereignis kennzeichnendes Signal nur dann von der Steuerleitung 76.2 zur Steuerleitung 78 weitergeleitet und somit der AV-Intervalltimer 34 ausgelöst, wenn der PMT-Schutzinhibitor 52 nicht auf Grund eines an der Steuerleitung 62 anliegenden Sperrsignals gesperrt ist.

Der Fernfeldschutzintervalltimer 38 wird ausgelöst, wenn über die Steuerleitung 16 ein ventrikuläres Sense-Ereignis kennzeichnendes Signal oder über die Steuerrückleitung 72 ein ventrikuläres Stimulationsereignis kennzeichnendes Signal eintreffen. In diesem Fall wird ein Fernfeldschutzintervall gestartet, währenddessen über die Steuerleitung 60 ein Sperrsignal an den Fernfeldschutzinhibitor 50 abgegeben wird, so dass ein ggf. an der Steuerleitung 74 anliegendes, atriales Sense-Signal nicht zur Steuerleitung 76.1 weitergeleitet wird.

Der PMT-Schutzintervalltimer 40 wird nach ventrikulären Stimulationsereignissen mit Ablauf des Fernfeldschutzintervalls durch ein Signal über eine Steuerleitung 80 gestartet, falls das Fernfeldschutzintervall durch ein ventrikuläres Stimulationsereignis ausgelöst wurde, also ein Steuersignal über die Steuerleitung 72 abgegeben wurde. Der Fernfeldschutzintervall wird somit nur ausgelöst, wenn im vorgegebenen zeitlichen Abstand zunächst ein Signal über die Steuerleitung 72 und anschließend ein Signal über die Steuerleitung 80 bei der PMT-Schutzintervalleinheit 40 eintreffen.

Der PMT-Schutzintervalltimer 40 wird außerdem nach dem Auftreten ventrikulärer Extrasystolen oder vorzeitiger ventrikulärer Kontraktionen mit Ablauf des Fernfeldschutzintervalls ebenfalls durch ein Signal über die Steuerleitung 80 gestartet, falls nach Auslösen des entsprechenden Fernfeldschutzintervalls ein Steuersignal über eine Steuerleitung 82 eintrifft. Dieses Steuersignal über die Steuerleitung 82 kennzeichnet das das Fernfeldschutzintervall auslösende und somit den Fernfeldschutztimer 38 startende ventrikuläre Ereignis als VES oder PVC. Das Signal über die Steuerleitung 82 stammt von der V-Sense-Klassifikationseinheit 48.

Im Ergebnis startet jedes atriale Sense-Ereignis, welches nicht innerhalb bereits gestarteter Intervalle wie der atrialen Refraktärzeit, dem Fernfeldschutzintervall und dem PMT-Schutzintervall auftritt, den AV-Intervalltimer 34, da in diesen Fällen der atriale Refraktärzeittimer 30, der Fernfeldschutzinhibitor 50 und der PMT-Schutzinhibitor 52 nicht sperrten. Wird der AV-Intervalltimer 34 durch ein, ein atriales Sense-Ereignis kennzeichnendes Steuersignal über die Steuerleitung 78 gestartet, beginnt ein AV-Intervall, welches mit seiner Dauer angibt, wann auf eine atriale Kontraktion spätestens eine ventrikuläre Kontraktion folgen sollte, damit sich eine effektive Pumpleistung des Herzens ergibt.

Der AV-Intervalltimer 34 wird nicht nur durch spontane atriale Ereignisse, also atriale Sense-Ereignisse, über die Steuerleitung 78 gestartet, sondern auch mit Abgabe eines atrialen Stimulationsimpulses nach Ablauf eines weiter unten erläuterten AA-Intervalls oder eines VA-Intervalls. Hierzu ist der AV-Intervalltimer 34 über Steruleitungen 64.1 und 66.1 mit dem Ausgang des entsprechenden AA-Timers 32 bzw. dem Ausgang des VA-Timers 42 verbunden.

Falls innerhalb des AV-Intervalls ein ventrikuläres Sense-Ereignis von der ventrikulären Detektionseinheit 12 erfasst wird, empfängt der AV-Intervalltimer 34 ein Signal über die Steuerleitungen 16, 16.1 und 16.2. Durch ein solches Steuersignal über die Leitung 16.2 wird der AV-Intervalltimer zurückgesetzt.

Empfängt der AV-Intervalltimer 34 während eines laufenden AV-Intervalls kein Steuersignal über die Leitung 16.2, wird er nicht zurückgesetzt und gibt mit Ablauf des vorgegebenen AV-Intervalls ein Steuersignal über die Steuerleitung 68 aus, mit dem die ventrikuläre Stimulationssteuereinheit 46 veranlasst wird, die ventrikuläre Stimulationseinheit 26 zu starten. Im Falle einer erfolgreichen Stimulation des Ventrikels gibt die ventrikuläre Stimulationssteuereinheit 46 über die Steuerrückleitung 72 ein Signal an den AV-Intervalltimer 34 ab, mit dem das die entsprechende Stimulation auslösende Steuersignal auf der Steuerleitung 68 gelöscht wird.

Eine Besonderheit der Steuereinheit 18 besteht in der AV-Überwachungseinheit 36 und deren Zusammenspiel mit der V-Sense-Klassifikationseinheit 48. Die AV-Überwachungseinheit 36 startet ein AV-Überwachungsintervall mit jedem über die Steuerleitung 76.3 einteffenden, ein atriales Sense-Ereignis kennzeichnenden außerhalb der atrialen Refraktärzeit und des Fernfeldschutzintervalls. Während der Dauer des auf diese Weise gestarteten AV-Überwachungsintervalls gibt die AV-Überwachungseinheit 36 über eine Steuerleitung 84 ein Signal an die V-Sense-Klassifikationseinheit 48 ab.

Neben dem Steuersignal von der AV-Überwachungseinheit 36 über die Steuerleitung 84 empfängt die V-Sense-Klassifikationseinheit 48 auch von der ventrikulären Detektionseinheit 12 abgegebene Signale im Falle der Detektion ventrikulärer Sense-Ereignisse. Falls die V-Sense-Klassifikationseinheit 48 ein solches, ein ventrikuläres Sense-Ereignis kennzeichnendes Signal über die Leitung 16.1 empfängt, während gleichzeitig auf der Steuerleitung 84 ein Ausgangssignal der AV-Überwachungseinheit 36 anliegt - falls also das ventrikuläre Sense-Ergeignis in das AV-Überwachungsintervall fällt -, gibt die V-Sense-Klassifikationseinheit 48 über die Steuerleitungen 82 und 86 Signale an den PMT-Schutzintervalltimer 40 bzw. den VA-Intervalltimer 42 ab.

Die AV-Überwachungseinheit 36 wird durch jedes ventrikuläre Sense- oder Stimulations-Ereignis zurückgesetzt, d. h., das jeweils entsprechende AV-Überwachungsintervall abgebrochen. Ein ein ventrikuläres Sense-Ereignis kennzeichnendes Signal empfängt die AV-Überwachungseinheit hierzu über eine Steuerleitung 16.3, während ein eine erfolgreiche ventrikuläre Stimulation kennzeichnendes Signal über eine Steuerleitung 72.2 eintrifft. Somit bewirken Signale über die Steuerleitungen 16.3 oder 72.2 jeweils einen Reset der AV-Überwachungseinheit 36 und damit einen Abbruch des jeweiligen AV-Überwachungsintervalls.

Bereits erwähnt wurde, dass die V-Sense-Klassifikationseinheit 48 ausgebildet ist, außerhalb eines jeweiligen AV-Überwachungsintervalls auftretende ventrikuläre Sense-Ereignisse als ventrikuläre Extrasystolen (VES) oder vorzeitige ventrikuläre Kontraktion (PVC) zu klassifizieren und in solchem Fall ein Steuersignal über die Steuerleitung 82 auszugeben.

Wird das ventrikuläre Sense-Ereignis hingegen durch die V-Sense-Klassifikationseinheit 48 als reguläres ventrikuläres Sense-Ereignis klassifiziert, welches nicht in ein laufendes AV-Intervall fällt, gibt die V-Sense-Klassifikationseinheit 48 über die Steuerleitung 86 ein Signal an den VA-Intervalltimer 42 ab. Zum Unterdrücken der Abgabe eines Ausgangssignals der V-Sense-Klassifikationseinheit 48 über die Steuerleitung 86 während eines laufenden AV-Intervalls ist die V-Sense-Klassifikationseinheit 48 über eine Steuerleitung 85 mit dem AV-Intervalltimer 34 verbunden. Somit wird der VA-Intervalltimer 42 durch ein Ausgangssignals der V-Sense-Kiassifikationseinheit 48 gestartet, wenn nach einem atrialen Signal, das in ein PMT-Schutzintervall fällt und das AV-Überwachungsintervall startet, ein ventrikuläres Signal in das AV-Überwachungsintervall fällt.

Wenn in einem definierten Zeitraum vor dem Eintreffen eines Signals über die Steuerleitung 86 ein ein atriales Sense-Ereignis kennzeichnendes Signal über die Steuerleitung 76.4 bei dem Intervalltimer 42 eingetroffen ist, welches in ein PMT-Schutzintervall fällt, gekennzeichnet durch ein Signal, welches über eine Steuerleitung an dem VA-Intervalltimer 42 anliegt, startet der VA-Intervalltimer 42 ein VA-Intervall und gibt gleichzeitig ein Steuersignal über eine Steuerleitung 88 an den AA-Intervalltimer 32 ab, welches diesen zurücksetzt. Das auf diese Weise gestartete VA-Intervall kann durch ein weiteres innerhalb dieses VA-Intervall eintreffendes Steuersignal über die Steuerleitung 76.4 zurückgesetzt werden. Gleichzeitig wird auch der AA-Intervalltimer 32 neu gestartet.

Zusammengefasst ist die Voraussetzung für das Starten eines VA-Intervalls durch den VA-Intervalltimer 42, das in definiertem zeitlichen Zusammenhang zunächst ein ein atriales Sense-Ereignis kennzeichnendes Signal über die Steuerleitung 76.4 bei dem VA-Intervalltimer 42 eintrifft, während gleichzeitig ein den Lauf eines PMT-Schutzintervalls kennzeichnendes Steuersignal über die Steuerleitung 62.1 bei dem VA-Intervalltimer 42 anliegt und ein, ein natürliches ventrikuläres Sense-Ereignis kennzeichnendes Signal über die Steuerleitung 86 folgt.

Läuft das VA-Intervall in dem VA-Intervalltimer 42 ohne vorhergehenden Reset ab, wird zum Ende des VA-Intervalls ein Steuersignal über die Steuerleitung 66 an die atriale Stimulationssteuereinheit 44 ausgegeben, die darauf über ein Signal über die Steuerleitung 20 die atriale Stimulationseinheit 24 startet und eine atriale Stimulation auslöst.

Der AA-Intervalltimer 32 wird ebenfalls durch ein ein atriales Sense-Ereignis kennzeichnendes Signal über die Steuerleitung 76.1 gestartet. Trifft innerhalb dieses AA-Intervalls kein weiteres Steuersignal über die Steuerleitung 76.1 oder über die Steuerleitung 88 ein, welche den AA-Intervalltimer zurücksetzen und ein neues AA-Intervall auslösen, gibt der AA-Intervalltimer 32 über die Steuerleitung 64 ein Steuersignal an die atriale Stimulationssteuereinheit 44 ab, die daraufhin die atriale Stimulationseinheit 24 auslöst. Im Falle erfolgreicher Stimulation und entsprechend erfolgreicher Capture-Control gibt die atriale Stimulationssteuereinheit 44 über die Steuerrückleitung 70 ein Signal an den AA-Intervalltimer 32 ab, welches ebenfalls ein Zurücksetzen des jeweils laufenden AA-Intervalls und des Starten eines neuen AA-Intervalls bewirkt.

Nicht in der Figur 1 dargestellt ist eine ebenfalls im Bereich der Erfindung liegende Variante der Steuereinheit 18 für einen VDD-Herzschrittmacher, der keine atriale Stimulationseinheit umfasst. Bei einem solchen VDD-Schrittmacher können somit die atriale Stimulationseinheit 24, die atriale Stimulationssteuereinheit 44, der VA-Intervalltimer und ggf. auch der AA-Intervalltimer wegfallen. Auch bei einem VDD-Schrittmacher sind jedoch in der Regel sowohl ein AA-Timer 32 als auch ein VA-Intervalltimer 42 vorgesehen, deren jeweilige Ausgangssignale wie zuvor beschrieben dem Starten des AV-Intervalltimers 34 über die Steuerleitungen 64.1 bzw. 66.1 dienen.

Ebenfalls nicht in der Figur 1 dargestellt ist eine Variante, bei der der jeweilige Schrittmacher eine Statistikeinheit umfasst. In solch einem Falle ist die V-Sense-Klassifikationseinheit vorzugsweise mit dieser Statistikeinheit verbunden, sodass mit der Statistikeinheit das Auftreten von der V-Sense-Klassifikationseinheit klassifizierte ventrikuläre Extrasystolen oder vorzeitiger ventrikulärer Kontraktionen erfasst und verarbeitet werden kann.

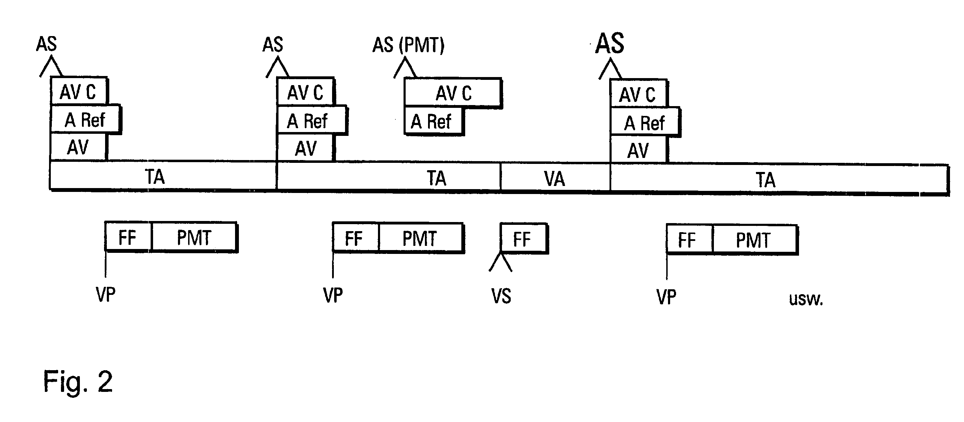

Zur Verdeutlichung dieses Verhaltens ist in Figur 2 beispielhaft der Ablauf der verschiedenen Intervalle in Abhängigkeit entsprechend atrialer Senseund ventrikulärer Sense- und Stimulatinsereignisse dargestellt. Atriale Sense-Ereignisse sind mit AS bezeichnet, ventrikuläre Sense-Ereignisse mit VS und ventrikuläre Stimulationsereignisse mit VP. Das AV-Überwachungsintervall ist mit AVC gekennzeichnet, die atriale Refraktärzeit mit ARef und das AV-Intervall mit AV. Außerdem ist das Fernfeldschutzintervall mit FF und das PMT-Schutzintervall mit PMT bezeichnet.

Ein Timing wie es in Figur 2 dargestellt ist, wird durch die Steuereinheit 18 aus Figur 1 bewirkt.

## Patentansprüche

1. Zweikammer-Herzschrittmacher, mit einer atrialen Detektionseinheit (10), die zum Erfassen elektrischer Signale im Atrium eines Herzens als atriale Sense-Ereignisse ausgebildet ist, einer ventrikulären Detektionseinheit (12), die zum Erfassen elektrischer Signale eines Ventrikels des Herzens als ventrikuläre Sense-Ereignisse ausgebildet ist, einer Stimulationseinheit, die zumindest zum Auslösen der Abgabe elektrischer Impulse an den Ventrikel als ventrikuläre Stimulationsereignisse ausbildet ist, sowie einer Steuereinheit, die mit der atrialen und der ventrikulären Detektionseinheit (10 und 12) sowie der Stimulationseinheit (24 und 26) verbunden und ausgebildet ist, das Auslösen der Impulsabgabe in Abhängigkeit von der atrialen Detektionseinheit (10) und der ventrikulären Detektionseinheit (12) so zu steuern, dass eine Impulsabgabe an den Ventrikel nach Ablauf einer AV-Zeit nach Erfassen eines atrialen Sense-Ereignisses ausgelöst wird, falls vor Ablauf der AV-Zeit kein ventrikuläres Sense-Ereignis durch die ventrikuläre Detektionseinheit (12) erfasst wird, wobei die Steuereinheit weiterhin ausgebildet ist ventrikuläre Sense-Ereignisse in mindestens zwei Kategorien einzuteilen, von denen eine erste Kategorie einer natürlichen Reizleitung vom Atrium zum Ventrikel zuzuordnende ventrikuläre Sense-Ereignisse betrifft, während eine zweite Kategorie ventrikulären Extrasystolen oder vorzeitigen ventrikulären Kontraktionen (PVC) zuzuordnende ventrikuläre Sense-Ereignisse betrifft und die Steuerung des Herzschrittmachers danach differenbziert wird, ob ein ventrikuläres Sense-Ereignis der ersten Kategorie zugeordnet wird, oder nicht, **dadurch gekennzeichnet, dass** für die Steuerung des Auslösens der Impulsabgabe ein AV-Überwachungsintervall vorgesehen ist, derart, dass das AV-Überwachungsintervall von jedem atrialen Sense-Ereignis ausgelöst wird, welches außerhalb einer atrialen Refraktärzeit liegt, wobei der Herzschrittmacher ausgebildet ist, innerhalb des AV-Überwachungsintervalls auftretende ventrikuläre Ereignisse der ersten Kategorie zuzuordnen.

2. Herzschrittmacher nach Anspruch 1, **dadurch gekennzeichnet, dass** das AV-Überwachungsintervall bei Auftreten eines ventrikulären Sense oder Stimulationsereignisses vor Ablauf einer vorgegebenen Zeitdauer des AV-Überwachungsintervalls vorzeitig beendet wird.

3. Herzschrittmacher nach Anspruch 1, **dadurch gekennzeichnet, dass** der Herzschrittmacher, insbesondere dessen Steuereinheit (18), ausgebildet ist, im Falle innerhalb des AV-Überwachungsintervalls und außerhalb des AV-Intervalls auftretender ventrikulärer Sense-Ereignisse ein VA-Intervall zu starten, welches vorzeitig beendet wird, falls vor dessen Ablauf ein atriales Sense-Ereignis auftritt.

4. Herzschrittmacher nach Anspruch 3, **dadurch gekennzeichnet, dass** der Herzschrittmacher, insbesondere dessen Steuereinheit (18), ausgebildet ist, mit einem atrialen Sense-Ereignis ein AA-Intervall zu starten, welches vorzeitig beendet wird, falls innerhalb des AV-Überwachungsintervalls ein ventrikuläres Sense-Ereignis auftritt und das VA-Intervall gestartet wird, oder falls vor Ablauf des AA-Intervalls ein atriales Sense-Ereignis auftritt.

5. Herzschrittmacher nach Anspruch 3 oder 4, mit einer atrialen Stimulationseinheit (24), die zum Auslösen der Abgabe elektrischer Impulse an das Atrium als atriale Stimulationsereignisse ausgebildet ist, **dadurch gekennzeichnet, dass** der Herzschrittmacher, insbesondere dessen Steuereinheit (18), ausgebildet ist, die Abgabe eines atrialen Stimulationsimpulses mit Ablauf des VA-Intervalls oder des AA-Intervalls auszulösen, falls das VA-Intervall oder dass AA-Intervall nicht vorzeitig beendet werden.

6. Herzschrittmacher nach Anspruch 1, **dadurch gekennzeichnet, dass** für die Steuerung des Auslösens der Impulsabgabe ein Fernfeldschutzintervall und sowie eine PMT-Schutzintervall vorgesehen sind,
- derart, dass das Fernfeldschutzintervall von jedem ventrikulären Stimulationserereignis und von jedem von der ventrikulären Detektionseinheit (12) erfassten ventrikulären Sense-Ereignis ausgelöst wird, wobei das Fernfeldschutzintervall hinsichtlich seiner Zeitdauer so bemessen ist, dass ein atriales Sense-Ereignis infolge einer Fernfeldwirkung eines ventrikulären Ereignisses in das Fernfeldschutzintervall fällt, und wobei das Fernfeldschutzintervall die Wirkung hat, dass während des Fernfeldschutzintervalls von der atrialen Detektionseinheit (10) erfasste atriale Ereignisse für das Auslösen der Impulsabgabe durch die Stimulationseinheit unberücksichtigt bleiben, so dass ein Auslösen der Impulsabgabe nach einem atrialen Sense-Ereignis infolge von Cross-Sensing oder Fernfelderfassung ventrikulärer Ereignisse unterbleibt sowie derart, dass das PMT-Schutzintervall von jedem ventrikulären Stimulationserereignis und von jedem, von der ventrikulären Detektionseinheit (12) erfassten ventrikulären Sense-Ereignis ausgelöst wird, wenn dieses ventrikuläre Ereignis außerhalb einer vorgebenen AV-Zeit oder außerhalb eines AV-Überwachungsintervalls erfasst wurde, wobei sich das PMT-Schutzintervall, falls es ausgelöst wurde, an das jeweilige Fernfeldschutzintervall anschließt und dieses verlängert und wobei das PMT-Schutzintervall die Wirkung hat, dass während des PMT-Schutzintervalls von der atrialen Detektionseinheit (10) erfasste atriale Ereignisse für das Auslösen der der Impulsabgabe durch die Stimulationseinheit unberücksichtigt bleiben, so dass ein Auslösen der Impulsabgabe infolge eines atrialen Ereignisses, welches auf eine retrograde Reizleitung vom Ventrikel zum Atrium zurückgeht, unterbleibt.

7. Herzschrittmacher nach Anspruch 1 und 6, **dadurch gekennzeichnet, dass** die Steuereinheit (18) ausgebildet ist, das AV-Überwachungsintervall nach jedem atrialen Sense-Ereignis auszulösen, welches außerhalb der atrialen Refraktärzeit und außerhalb des Fernfeldschutzintervalls liegt.

## Claims

1. A dual-chamber cardiac pacemaker, having an atrial detection unit (10), which is implemented for detecting electrical signals in the atrium of a heart as atrial sensing events, a ventricular detection unit (12), which is implemented for detecting electrical signals of a ventricle of the heart as ventricular sensing events, a stimulation unit, which is implemented at least to trigger the delivery of electrical pulses to the ventricle as ventricular stimulation events, as well as a control unit, which is connected to the atrial and the ventricular detection units (10 and 12) and to the stimulation units (24 and 26) and is implemented to control the triggering of the pulse delivery as a function of the atrial detection unit (10) and the ventricular detection unit (12) in such a way that a pulse delivery to the ventricle is triggered after expiration of an AV time after detecting an atrial sensing event if, before expiration of the AV time, no ventricular sensing event is detected by the ventricular detection unit (12), the control unit also being implemented to divide ventricular sensing events into at least two categories, of which a first category relates to ventricular sensing events to be assigned to a natural stimulus output from the atrium to the ventricle, while a second category relates to ventricular sensing events to be assigned to ventricular extrasystoles or premature ventricular contractions (PVC) and the control of the cardiac pacemaker is differentiated according to whether or not a ventricular sensing event is assigned to the first category, **characterized in that** an AV monitoring interval is provided for the control of the triggering of the pulse delivery in such a way that the AV monitoring interval is triggered by every atrial sensing event which lies outside an atrial refractory time, the cardiac pacemaker being implemented to assign ventricular events occurring within the AV monitoring interval to the first category.

2. The cardiac pacemaker according to Claim 1, **characterized in that** the AV monitoring interval is ended early in the event of the occurrence of a ventricular sensing or stimulation event before expiration of a predefined duration of the AV monitoring interval.

3. The cardiac pacemaker according to Claim 1, **characterized in that** the cardiac pacemaker, particularly its control unit (18), is implemented to start a VA interval in case of ventricular sensing events occurring within the AV monitoring interval and outside the AV interval, which is ended early if an atrial sensing event occurs before its expiration.

4. The cardiac pacemaker according to Claim 3, **characterized in that** the cardiac pacemaker, particularly its control unit (18), is implemented to start an AA interval upon an atrial sensing event, which is ended early if a ventricular sensing event occurs within the AV monitoring interval and the VA interval is started, or if an atrial sensing event occurs before expiration of the AA interval.

5. The cardiac pacemaker according to Claim 3 or 4, having an atrial stimulation unit (24) which is implemented to trigger the delivery of electrical pulses to the atrium as atrial stimulation events, **characterized in that** the cardiac pacemaker, particularly its control unit (18), is implemented to trigger the delivery of an atrial stimulation pulse upon expiration of the VA interval or the AA interval if the VA interval or the AA interval is not ended early.

6. The cardiac pacemaker according to Claim 1, **characterized in that** a far field protective interval and a PMT protective interval are provided for the control of the triggering of the pulse delivery,
- in such a way that the far field protective interval is triggered by every ventricular stimulation event and by every ventricular sensing event detected by the ventricular detection unit (12), the far field protective interval being dimensioned in regard to its duration in such a way that an atrial sensing event as a result of a far field effect of a ventricular event falls in the far field protective interval, and the far field protective interval having the effect that atrial events detected by the atrial detection unit (10) during the far field protective interval remain unconsidered for the triggering of the pulse delivery by the stimulation unit, so that triggering of the pulse delivery after an atrial sensing event as a result of cross sensing or far field detection of ventricular events is suppressed, and in such a way that the PMT protective interval is triggered by every ventricular stimulation event and by every ventricular sensing event detected by the ventricular detection unit (12) if this ventricular event was detected outside a predefined AV time or outside an AV monitoring interval, the PMT protective interval, if it is triggered, following the particular far field protective interval and extending it and the PMT protective interval having the effect that atrial events detected by the atrial detection unit (10) during the PMT protective interval remain unconsidered for the triggering of the pulse delivery by the stimulation unit, so that triggering of the pulse delivery as a result of an atrial event which originates from a retrograde stimulus output from the ventricle to the atrium is suppressed.

7. The cardiac pacemaker according to Claims 1 and 6, **characterized in that** the control unit (18) is implemented to trigger the AV monitoring interval after every atrial sensing event which lies outside the atrial refractory time and outside the far field protective interval.

## Revendications

1. Pacemaker à deux chambres, comprenant une unité de détection atriale (10), qui est conçue pour enregistrer des signaux électriques dans l'atrium d'un coeur en tant qu'événements de sens atriaux, une unité de détection (12) ventriculaire, qui est conçue pour enregistrer des signaux électriques d'un ventricule du coeur en tant qu'événements de sens ventriculaires, une unité de stimulation, qui est conçue au moins pour déclencher l'envoi d'impulsions électriques au ventricule en tant qu'événements de stimulation ventriculaires, ainsi qu'une unité de commande, qui est reliée à l'unité de détection atriale et à l'unité de détection ventriculaire (10 et 12) ainsi qu'à l'unité de stimulation (24 et 26) et est conçue pour commander le déclenchement de l'envoi d'impulsion en fonction de l'unité de détection (10) atriale et de l'unité de détection (12) ventriculaire de telle sorte qu'un envoi d'impulsion au ventricule est déclenché après l'expiration d'une période AV après l'enregistrement d'un événement de sens atrial, dans le cas où aucun événement de sens ventriculaire n'est enregistré par l'unité de détection (12) ventriculaire avant l'expiration de la période AV, l'unité de commande étant conçue également pour diviser les événements de sens ventriculaires en au moins deux catégories, dont une première catégorie concerne des événements de sens ventriculaires à attribuer à une conduction naturelle de l'atrium au ventricule, alors qu'une seconde catégorie concerne les événements de sens ventriculaires à attribuer à des extrasystoles ventriculaires ou des contractions ventriculaires prématurées (PVC) et la commande du pacemaker différencie si un événement de sens ventriculaire est attribué à la première catégorie ou non, **caractérisé en ce que**, pour la commande du déclenchement de l'envoi d'impulsion, il est prévu un intervalle de contrôle AV de telle sorte que l'intervalle de contrôle AV est déclenché par tout événement de sens atrial qui se situe en dehors d'une période réfractaire atriale, le pacemaker étant conçu pour attribuer des événements ventriculaires apparaissant à l'intérieur de l'intervalle de contrôle AV à la première catégorie.

2. Pacemaker selon la revendication 1, **caractérisé en ce que** l'intervalle de contrôle AV est achevé de façon prématurée en cas d'apparition d'un événement de sens ou de stimulation ventriculaire avant l'expiration d'une durée prédéfinie de l'intervalle de contrôle AV.

3. Pacemaker selon la revendication 1, **caractérisé en ce que** le pacemaker, en particulier son unité de commande (18), est conçu pour démarrer un intervalle VA dans le cas d'événement de sens ventriculaires survenant à l'intérieur de l'intervalle de contrôle AV et à l'extérieur de l'intervalle AV, lequel intervalle est achevé de façon prématurée si un événement de sens atrial arrive avant son expiration.

4. Pacemaker selon la revendication 3, **caractérisé en ce que** le pacemaker et en particulier son unité de commande (18), est conçu pour démarrer un intervalle AA avec un événement de sens atrial qui est terminé de façon prématurée dans le cas où un événement de sens ventriculaire apparaît à l'intérieur de l'intervalle de contrôle AV et où l'intervalle VA est démarré, ou dans le cas où un événement de sens atrial apparaît avant l'expiration de l'intervalle AA.

5. Pacemaker selon la revendication 3 ou 4, avec une unité de stimulation (24) axiale, qui est conçue pour déclencher l'envoi d'impulsions électriques à l'atrium sous la forme d'événements de stimulation atriaux, **caractérisé en ce que** le pacemaker, en particulier son unité de commande (18), est conçu pour déclencher l'envoi d'une impulsion de stimulation atriale avec l'expiration de l'intervalle VA ou de l'intervalle AA, dans le cas où l'intervalle VA ou l'intervalle AA n'est pas achevé de façon prématurée.

6. Pacemaker selon la revendication 1, **caractérisé en ce que** un intervalle de protection de champ éloigné et un intervalle de protection PMT sont prévus pour la commande du déclenchement de l'envoi d'impulsion,
- telle sorte que l'intervalle de protection de champ éloigné est déclenché par tout événement de stimulation ventriculaire et par tout événement de sens ventriculaire enregistré par l'unité de détection ventriculaire (12), l'intervalle de protection de champ éloigné étant conçu en ce qui concerne sa durée de telle sorte qu'un événement de sens atrial tombe du fait d'un effet de champ éloigné d'un événement ventriculaire dans l'intervalle de protection de champ éloigné, et l'intervalle de protection de champ éloigné ayant pour effet que des événements atriaux enregistrés par l'unité de détection atriale (10) pendant l'intervalle de protection de champ éloigné ne sont pas pris en compte pour le déclenchement de l'envoi d'impulsion par l'unité de stimulation, de sorte qu'un déclenchement de l'envoi d'impulsion après un événement de sens atrial en raison du Cross-Sensing ou de l'enregistrement de zone éloignée d'événements ventriculaires est supprimé de telle sorte que l'intervalle de protection PMT est déclenché par tout événement de stimulation ventriculaire et par tout événement de sens ventriculaire enregistrés par l'unité de détection ventriculaire (12), lorsque cet événement ventriculaire est enregistré en dehors d'une période AV prédéfinie ou en dehors d'un intervalle de contrôle AV, l'intervalle de protection PMT, s'il a été déclenché, faisant suite à l'intervalle de protection de champ éloigné concerné et prolongeant celui-ci et l'intervalle de protection PMT ayant pour effet que des événements atriaux enregistrés pendant l'intervalle de protection PMT par l'unité de détection (10) atriale ne sont pas pris en compte pour le déclenchement de l'envoi d'impulsion par l'unité de stimulation, de sorte qu'un déclenchement de l'envoi d'impulsion dû à un événement atrial, qui remonte à une conduction rétrograde du ventricule vers l'atrium, est supprimé.

7. Pacemaker selon les revendications 1 à 6, **caractérisé en ce que** l'unité de commande (18) est conçue pour déclencher l'intervalle de contrôle AV après chaque événement de sens atrial, qui se situe en dehors de la période réfractaire atriale et en dehors de l'intervalle de protection de zone éloignée.
